# FASCICULE DE BREVET EUROPEEN

(11) **EP 1 278 831 B1**
(45) Date de publication et mention de la délivrance du brevet: **30.11.2005**
(21) Numéro de dépôt: 01921457.6
(22) Date de dépôt: 03.04.2001
(51) Int. Cl.: C12N 15/10, C07H 21/04, G01N 27/447

(54) **PROCEDE D'EXTRACTION INDIRECTE D'ADN D'ORGANISMES NON CULTIVABLES**
VERFAHREN ZUR INDIREKTEN ISOLATION VON DNS AUS NICHT-KULTIVIERBAREN MIKROORGANISMEN
METHOD FOR INDIRECTLY EXTRACTING NON-CULTIVABLE DNA ORGANISMS

(30) Priorité: 26.04.2000 FR 0005274
(43) Date de publication de la demande: 29.01.2003
(73) Titulaire: Libragen, 69300 Caluire et Cuire (FR)
(72) Inventeur: NALIN, Renaud, F-69300 Caluire et Cuire (FR); ROBE, Patrick, F-69340 Francheville (FR); TRAN VAN, Van, F-69100 Villeurbanne (FR)
(74) Mandataire: Vuillermoz, Bruno
(86) Numéro de dépôt international: PCT/FR2001/000992
(87) Numéro de publication internationale: WO 2001/081357

(56) Documents cités:
- EP-A- 0 388 053
- EP-A- 0 512 181
- WO-A-94/22904
- WO-A-98/17685
- WO-A-99/08532
- AMARAL JOHN A ET AL: "Atmospheric methane consumption by forest soils and extracted bacteria at different pH values." APPLIED AND ENVIRONMENTAL MICROBIOLOGY, vol. 64, no. 7, juillet 1998 (1998-07), pages 2397-2402, XP002156716 ISSN: 0099-2240
- ROEMLING U ET AL: "THE IMPACT OF TWO-DIMENSIONAL PULSED-FIELD GEL ELECTROPHORESIS TECHNIQUES FOR THE CONSISTENT AND COMPLETE MAPPING OF BACTERIAL GENOMES REFINED PHYSICAL MAP OF PSEUDOMONAS-AERUGINOSA PAO" NUCLEIC ACIDS RESEARCH, vol. 19, no. 12, 1991, pages 3199-3206, XP002180817 ISSN: 0305-1048
- SUAU ANTONIA ET AL: "Direct analysis of genes encoding 16S rRNA from complex communities reveals many novel molecular species within the human gut." APPLIED AND ENVIRONMENTAL MICROBIOLOGY, vol. 65, no. 11, novembre 1999 (1999-11), pages 4799-4807, XP002156717 ISSN: 0099-2240 cité dans la demande
- ROCHELLE P A ET AL: "A SIMPLE TECHNIQUE FOR ELECTROELUTION OF DNA FROM ENVIRONMENTAL SAMPLES" BIOTECHNIQUES, vol. 11, no. 6, 1991, pages 724, 726-728, XP000971960 ISSN: 0736-6205
- GRIESS GARY A ET AL: "Unlimited increase in the resolution of DNA ladders." ELECTROPHORESIS, vol. 21, no. 5, mars 2000 (2000-03), pages 859-864, XP001030781 ISSN: 0173-0835

## Description

L'invention concerne un procédé d'extraction indirecte d'ADN, en particulier de fragments d'ADN de taille supérieure à 300 kB, d'organismes non cultivables contenus dans un échantillon environnemental. Elle se rapporte également à l'ADN, en particulier celui de taille supérieure à 300 kB, susceptible d'être obtenu par le procédé, de même qu'à une librairie d'ADN constituée des fragments d'ADN extraits par ledit procédé.

Dans le cadre de la recherche de molécules actives, et notamment de nouveaux antibiotiques, acides aminés, enzymes, vitamines, on propose différentes méthodes, dont le principe repose sur le criblage des métabolites produits par des organismes provenant de différents environnements. Ainsi, des produits de toute première importance avec des activités antibiotiques, anticancéreuses ou pesticides ont été isolés à partir de microorganismes comme par exemple *Streptomyces, Nocardia* ou *Actinoplanes* etc...

Le sol et les sédiments constituent des environnements majeurs pour la recherche de nouveaux métabolites actifs, non seulement de par la quantité très importante de micro-organismes qu'ils contiennent mais également en raison de l'importante diversité de ces micro-organismes. On sait que le sol peut contenir de 10⁹ à 10¹¹ bactéries par gramme, dont 1 % au maximum sont cultivables sur milieux synthétiques (Voir AMANN R. I., Ludwig W., Schleifer KH (1995) " Phylogenetic identification and *in situ* détection of individual microbial cells without cultivate " Microbiological review vol 59 N° 11 143-169; Whitman WB, Coleman DC., Wiebe WJ. 1998. Prokaryotes: the unseen majority Proceeding National Academy of Science USA, Vol 95: 6578-6583).

Par ailleurs on évalue de 1000 à 10 000 le nombre d'espèces bactériennes par gramme de sol. En effet, par des techniques de réassociation ADN-ADN les résultats indiquent que la diversité génétique des microorganismes est certainement supérieure à 4000 espèces par échantillon de sol (Torsvik et al. 1990, Applied Environemental Microbiology, 56:782-787). Toujours basé sur des méthodologies n'impliquant pas la culture *in vitro* des microorganismes, Torsvik V, Goksoyr J., Daae FL., Sorheim R., Michalsen J., Salte K. 1994, p.39-48 In Beyond the Biomass, K. Ritz, J. Dighton and K.E. Giller (eds), John Wiley and Sons, Chichester indiquent que la diversité des espèces bactériennes peut atteindre 13000 espèces pour 100g de sol. Ainsi, en estimant à 10¹⁰ cellules bactériennes par gramme de sol, une espèce bactérienne doit être représentée par une moyenne de 10⁶ cellules, même pour les espèces les plus rares.

Par ailleurs, les bactéries ont été détectées dans un grand nombre d'environnement allant de la stratosphère jusqu'au fin fond des abysses, incluant les milieux les plus extrêmes en termes de conditions physico-chimiques. Ainsi, la diversité bactérienne s'explique par une diversité de localisation des populations de bactéries, tout d'abord au niveau des macro-environnements qu'elles ont colonisés, mais aussi au niveau des micro-environnements qui caractérisent par exemple et de façon non limitative la structuration des sols comme décrit par Ranjar L., Poly F., Combrisson J, richaume A., Nazaret S. 1998. A single procédure to recover DNA from the surface or inside aggregates and in various fractions of soil suitable for PCR-based assays of bacterial communities. European Journal of Soil Biology, 34 (2), 89-97.

Un grand nombre d'études portant sur la diversité des bactéries, basées sur l'analyse des gènes d'ADN ribosomique ADNr16S, révèlent la présence de nombreux nouveaux phylums appartenant aux domaines des Archaebactéries mais aussi des bactéries. Le nombre de divisions bactériennes identifiées a triplé en 10 ans. Ainsi, l'énorme diversité des bactéries du sol est encore inconnue. Elle est peu considérée dans les travaux de recherche scientifique et n'est pas accessible et donc pas exploitée au niveau industriel.

L'un des objectifs poursuivis est d'accéder à cet énorme potentiel en extrayant l'ADN des 99 % de bactéries non cultivables restantes.

Pour ce faire, une première solution consiste à améliorer les milieux de cultures en augmentant les connaissances sur la physiologie des bactéries de sorte à les rendre cultivables et donc diminuer le nombre de bactéries non cultivables. Une telle technique est mise en oeuvre depuis l'origine de la microbiologie et a donné de très bons résultats. En effet, près de 5000 microorganismes ont été décrits, tout environnement confondu. Aussi, plus de 40 000 molécules ont été caractérisées et près de la moitié présentent une activité biologique. Néanmoins, une telle technique présente l'inconvénient d'être relativement lente et fastidieuse.

Une autre solution consiste à extraire directement l'ADN des organismes non cultivables à partir de différents environnements par lyse chimique et/ou enzymatique tel que décrit par exemple dans les documents US-A-5 824 485, WO 97/12991, ou encore SUAU ANTONIA et al " Direct analysis of genes encoding 16S rRNA from complex communities reveals many novel molecular species within the humain gut " APPLIED AND ENVIRONMENTAL MICROBIOLOGY, Vol 65, No 11, Novembre 1999, pages 4799 et 4807, et ROCHELLE P.S. et al " A simple technique for electroelution of DNA from environmental samples " BIOTECHIQUES, Vol 11, No 6, 1991, pages 724, 726-728. Les environnements considérés sont soit de type aquatique ce qui oblige une concentration des cellules bactériennes comme l'illustrent les travaux de Stein J.L., Marsh T.L., Wu K.Y., Shizuya H. and deLong E., 1996 - Characterization of uncultivated prokaryotes: isolation and alalysis of a 40-kilobas-pair genome fragment from a planktonic marine archaeon, Journal of Bacteriology, 178:591-599, soit terrestre.

L'un des principaux inconvénients que présente cette technique d'extraction directe est de conduire à l'extraction d'ADN uniquement de faible taille, de l'ordre de 1 à 23 kB au maximum. En effet, les contraintes physico-chimiques qui sont imposées à l'ADN au cours de ce type d'expérimentation conduisent à sa dégradation. Frostegard A., Courtois S. Ramisse V., Clerc S., Bernillon D., Le gall F., Jeannin P., Nesme X., Simonet P. 1999. Quantification of bias related to the extraction of DNA directly from soils. APPLIED AND ENVIRONMENTAL MICROBIOLOGY, VOL 65 (12): 5409-5420, montrent clairement que l'amélioration des rendements d'extraction d'ADN notamment par des techniques de broyage ou de sonication entraînent une forte dégradation de l'ADN récupéré.

De plus, l'ADN extrait comprend l'ADN extra cellulaire contenu dans le sol mais aussi l'ADN eucaryotique (champignons, cellules végétales, animales); procaryotique (bactéries) et les autres organismes présents dans le sol (protozoaires...). Il en résulte que les librairies d'ADN obtenues sont souvent fortement contaminées par des clones bactériens recombinants contenant de l'ADN non désiré (eucaryotique, ADN extracellulaire dégradé). Par ailleurs, les librairies ainsi constituées sont caractérisées par des inserts d'ADN de petite taille (inférieure à 30Kb). Ces librairies ne sont donc pas adaptées pour des applications telles que l'analyse de génomes ou de métagénomes complets ou encore la recherche, l'étude et l'exploitation de voies métaboliques complètes ou presque complètes.

Du fait de la dilution des ADNs cibles par les ADNs non-cibles, il est dès lors indispensable d'amplifier sélectivement la proportion de l'ADN cible par PCR pour constituer des librairies d'ADN qualifiées, comme décrit dans le document SUAU précité. Cette approche présente la principale contrainte de ne pouvoir accéder qu'à une information génétique similaire à celle déjà connue excluant de fait d'accéder à des séquences d'ADN totalement nouvelles et originales. Néanmoins, l'utilisation d'amorces PCR définies dans des régions très conservées a permis d'isoler des gènes d'intérêt appartenant à des bactéries non isolées comme décrit par exemple par Seow K., Meurer G., Gerlitz M., Wendt-Pienkowski E., Hutchinson C.R. and Davies J., 1997 - A study of itérative type II Polyketide Synthases, using bacterial genes cloned from soil DNA: a means to access and use genes from uncultured microorganisms., Journal of Bacteriology, 179:7360-7368.

D'autres méthodes de sélection ont été développées. Ces méthodes consistent à soumettre la communauté bactérienne à une pression de sélection permettant l'enrichissement de populations bactériennes déterminées. On espère ainsi accéder préférentiellement à l'information génétique recherchée. D'autres sélections se basant sur la composition de l'ADN (% GC) ou sa complexité ont également été décrites comme par exemple dans le document WO 99/45154.

Le problème que se propose de résoudre l'invention est donc de développer un procédé simple permettant d'extaire, de purifier, de séparer et d'extraire des fragments d'ADN de grande taille, en particulier supérieure à 300 kB, d'organismes non cultivables et principalement des bactéries. L'obtention de ces ADNs de grande taille ainsi purifiés et extraits, est une condition sine qua none à la réalisation de librairies d'ADN génomiques et métagénomiques qualifiées dans le champs d'application.

Un autre objectif de l'invention est de proposer un procédé qui permette de purifier, de séparer et d'extraire l'ADN cible exempt de toute contamination d'ADN, notamment eucaryote ou de l'ADN extracellulaire dégradé, en vue de la réalisation de librairies qualifiées.

Un autre problème que se propose de résoudre l'invention est de pouvoir appliquer le procédé développé sur de nombreux échantillons environnementaux, et notamment mais de façon non limitative, les végétaux, les insectes, par exemple les arthropodes, les mollusques, les éponges, les crustacés, les plathelmintes, les nématodes, les bioréacteurs tels que les biofilms, fermenteurs, boues activées et plus généralement les milieux aquatiques mais également des échantillons biologiques animaux (par exemple le lisier de porc, bol alimentaire du rumen) ou humains.

S'agissant de ce dernier environnement, le document EP-A-0 939 118 décrit une méthode d'extraction directe d'ADN et d'ARN à partir de fèces, selon laquelle la matière est mélangée en milieu détergent avant d'être soumise à l'extraction proprement dite de l'ADN et de l'ARN dans un solvant approprié. Aucune indication n'est donnée concernant la taille de l'ADN extrait mais on peut s'attendre à extraire des petits fragments. En outre et de même que précédemment, on assiste à une contamination de l'ADN cible par de l'ADN eucaryote.

L'objectif de l'invention est donc de proposer un procédé d'extraction indirecte de l'ADN cible d'organismes non cultivables contenus dans des d'échantillons environnementaux, permettant d'obtenir des fragments d'ADN spécifiques et de taille supérieure à 300 kB susceptibles d'être clonés par les techniques usuelles connues de l'homme du métier. De fait, cet ADN permettra la constitution de librairies d'ADN qualifiées offrant une meilleure représentativité des génomes ou des métagénomes considérés.

Pour ce faire, l'invention propose un procédé d'extraction indirecte de d'ADN cible de taille supérieure à 300 kB d'organismes non cultivables contenus dans un échantillon environnemental selon lequel :
- on broie en milieu liquide ledit échantillon ;
- on récupère le broyât obtenu ;
- on sépare les organismes du reste du broyât par sédimentation sur coussin de densité supérieure à 1 ;
- on récupère les organismes isolés à la surface du coussin que l'on inclut dans un bloc d'agarose ;
- on extrait l'ADN par lyse dans le bloc d'agarose ;
- on positionne le bloc dans un gel d'agarose que l'on soumet à au moins une électrophorèse en champs alternés,
- enfin, on récupère les brins d'ADN extraits.

Le Demandeur a constaté que la séparation préalable des organismes du reste du broyât par sédimentation sur coussin de densité, puis lyse de ces organismes directement dans un bloc d'agarose permettaient de séparer et d'obtenir, grâce à la mise en oeuvre d'une électrophorèse en champ pulsé, des fragments d'ADN de taille supérieure à 300 kB. En effet, cette façon de procéder permet de ne jamais manipuler directement l'ADN, évitant ainsi toute dégradation due à des phénomènes de contraintes mécaniques ou enzymatiques. De plus, le procédé de l'invention permet une purification sélective et importante des fragments d'ADN recherchés en s'affranchissant des inévitables pertes d'ADN liées aux méthodes classiques de purification (extractions organiques, précipitations, méthodes d'échange d'ion etc...). En outre, pour ne pas altérer l'intégrité des fragments d'ADN, particulièrement dans le cas de fragments d'ADN de grande taille ou ne pas affecter les efficiences de clonage ultérieur, les étapes de préparation des fragments d'ADN sont avantageusement effectuées sans intercalant (bromure d'éthidium etc...), ni de visualisation aux UVs.

S'agissant de l'étape de séparation des organismes du reste du broyat, le choix de la densité du coussin sera fonction de la densité de l'organisme qui doit être séparé des autres constituants du broyat. Ainsi, lorsqu'il s'agit de séparer la fraction bactérienne du reste des organites et débris, on sait que celle-ci présente, en fonction des espèces, une densité qui peut varier de 1 à 1,4. Selon les cas, la densité des coussins utilisés sera ajustée afin d'optimiser la séparation de la fraction bactérienne des autres constituants du broyat.

Dans un mode de réalisation préféré, on fera suivre l'étape de séparation des organismes par une étape de centrifugation sur gradient de densité isopycnique, permettant ainsi de séparer les organismes en fonction de leur densité.

Par ailleurs et selon une caractéristique avantageuse du procédé de l'invention, on accélère l'étape de sédimentation par centrifugation.

La technique d'électrophorèse en champ alterné est parfaitement connue de l'homme du métier et plus particulièrement décrite dans les document US-A-5 135 628 et dans la revue "1c technoscope de biofutur " N°127 d'octobre 1993, de sorte qu'il ne sera pas revenu sur son principe plus avant.

Avantageusement, on soumet les brins d'ADN séparés et purifiés à l'issue de l'étape d'électrophorèse en champs alternés (ci-après dénommée première migration électrophorétique), à une étape de restriction enzymatique suivie d'au moins une électrophorèse en champs alternés (seconde migration électrophorétique). Dans ce cas, la première migration électrophorétique est utilisée pour extraire les fragments d'ADN de grande taille et les séparer des débris cellulaires. En pratique, on découpe les zones de gel contenant les ADNs de taille souhaitée (par exemple supérieure à 300 kB) dans une approche préparative. Les fragments d'ADN inclus dans les blocs d'agarose ainsi obtenus sont alors soumis à une restriction enzymatique directement dans le bloc d'agarose ou éventuellement, après extraction de l'ADN de l'agarose. Les blocs d'agarose contenant les ADNs digérés sont alors de nouveau positionnés dans un gel d'agarose et sont alors soumis à au moins une migration electrophorétique permettant de séparer et purifier les ADNs digérés.

Dans un premier mode de réalisation, l'étape de restriction enzymatique est suivie de deux électrophorèse en champs alternés (respectivement seconde et troisième migration électrophorétique).

En pratique, les blocs d'agarose contenant les fragments d'ADN digérés sont positionnés dans un second gel de manière excentrée. Le gel est successivement positionné dans la cuve d'électrophorèse en champs alternés, dans un sens (seconde migration) puis dans le sens opposé (troisième migration). Au cours de la seconde migration électrophorétique de courte durée, le positionnement du gel dans un champs électrique homogène permet de faire sortir du gel les petits fragments d'ADN, en maintenant les grands fragments dans le gel. Au cours de la troisième migration, le gel est positionné dans l'autre sens et les grands fragments peuvent être séparés selon leur taille. Le demandeur a constaté que la double migration permettait d'éliminer les fragments de petite taille enchevêtrés dans les fragments d'ADN de la taille souhaitée.

Dans un second mode de réalisation, l'étape de restriction enzymatique est suivie de trois électrophorèses en champs alternés (respectivement seconde, troisième et quatrième migration électrophorétique).

En pratique, après la seconde migration électrophorétique une bande d'agarose contenant les fragments d'ADN digérés de la taille souhaitée est découpée et remise en place, en orientation inverse, dans un nouveau gel strictement identique. Ce gel est soumis à la même migration électrophorétique (troisième migration). La mobilité propre de chaque fragment d'ADN, en tous points de la zone d'agarose découpée, étant conservée entre la seconde et la troisième migration, tous les fragments d'ADN migrent in fine vers une même zone concentrée du gel. Le demandeur a constaté que les fragments d'ADN de petite taille enchevêtrés dans les fragments d'ADN de la taille souhaitée (contenu dans la zone de gel où l'ADN est concentré) pouvaient être éliminés en soumettant cette zone de gel ou ce gel à une quatrième migration électrophorétique en champs alternés selon une orientation identique à celle de la troisième migration. Cette dernière migration est caractérisée par des paramètres très peu séparatifs n'affectant que les petits fragments d'ADN.

Enfin et dans les deux modes de réalisation, on récupère les brins d'ADNs ainsi préparés (purification, digestion, sélection, concentration) par les techniques connues de l'homme du métier, telles que par exemple, électroélution (comme par les systèmes commercialisés par les société Biorad et Schleicher & Schuell), digestion de l'agarose (en utilisant les enzymes commercialisées comme la β-agarase commercialisée par New England Biolabs ou la gélase Epicentre Technologies et selon les instructions des fournisseurs), utilisation de kits commercialisés (Nucleo Trap DNA extraction Kit, Clontech; Gene Clean turbo spin BIO101) et connus de l'homme du métier.

En d'autres termes, la combinaison du broyage, de la sédimentation différentielle, de la lyse du bloc d'agarose et de la succession d'étapes d'électrophorèses en champs alternés, permet,
- de purifier l'ADN en éliminant tout débris inhérant à la lyse des cellules incluses dans le bloc,
- de séparer l'ADN en fonction de sa taille
- d'obtenir de l'ADN concentré à la taille souhaité

Autrement dit, le procédé développé dans l'invention permet non seulement de purifier, séparer et visualiser l'ADN par électrophorèse sur gel, mais aussi de récupérer cet ADN en contrôlant précisément la taille des ADNs requise pour les étapes ultérieures de clonage. L'ADN ainsi obtenu peut servir de matrice pour être cloné par des techniques classiques. Ces techniques peuvent utiliser soit la restriction enzymatique (bouts cohésifs), soit le clonage bouts francs ou encore la synthèse de queues homopolymériques complémentaires sur l'insert et le vecteur comme il est décrit dans Sambrook J., Fritsch EF., Maniatis T. 1989. Molecular Cloning: a laboratory manual II édition, Cold Spring Harbor laboratory, N.Y. L'inconvénient majeur que présente la restriction enzymatique, est la sélection engendrée par les sites de reconnaissances des enzymes de restriction utilisées. De fait, la restriction affecte inégalement les fragments d'ADN à taux GC variables. La ligation bouts francs et la synthèse de queues homopolymériques complémentaires permet d'éviter toute sélection des fragments d'ADN.

De plus, de nombreux vecteurs de clonage ou d'expression ont déjà été décrits dans l'art antérieur. Il s'agit de manière non limitative de plasmides largement commercialisés, des phagemides comme par exemple (Bluescript pSK), des vecteurs dérivés de phages comme par exemple ceux commercialisés par la société Stratagene (Lambda DASH II et ZAP II), des cosmides comme par exemple ceux commercialisés par Stratagene (SuperCos et pWE15) et Epicentre TEBU (pWED cosmid cloning kit), des fosmides comme celui décrit par Kim U.J., Shizuya H., de Jong P.J., Birren B. and Simon M.I., 1992 - Stable propagation of cosmid sized human DNA inserts in a F factor based vector, Nucleic Acids Research 20(5):1083-1085), des chromosomes artificiels PAC comme décrit par Ioannou P.A., Amemiya C.T., Games J., Kroisel P.M., Shizuya H., Chen C., Batzer M.A. and de Jong P., 1994 - a new bacteriophage P1-derived vector for the propagation of large human DNA fragments, Nature Genetics, 6:84-89, ; BAC comme décrit par Shizuya H., Bruce Birren, Ung-Jin Kim, Valeria Mancino, Tatiana Slepak, Yoshiaki Tachiiri, and Melvin I. Simon, 1992 - A bacterial cloning system for cloning large human DNA fragments., Proc. Natl. Acad. Sci., 89:8794-8797.) et YAC comme décrit par Larin Z., Monaco A.P. and Lehrach H., 1991 - Yeast artificial chromosome libraries containing large inserts from mouse and human DNA, Proc. Natl. Acad. Sci., 88:4123-412. Les vecteurs, techniques de préparation des vecteurs, les techniques de clonage ont fait l'objet de nombreux travaux et sont déjà largement décrits.

Comme déjà dit, un des objectifs du procédé de l'invention est qu'il puisse s'adapter à une grande variété d'environnements.

Dans la suite de la description et dans les revendications, par *"organisme non cultivable*", on désigne tout organisme non cultivable sur milieu synthétique tel que notamment, mais de façon non limitative, les procaryotes, tels que les bactéries (endosymbiotes ou parasites), les archébactéries, les protistes.

Bien entendu, le procédé de l'invention permet dans le même temps d'isoler les organismes cultivables dans la mesure où la sédimentation sur coussin ne permet pas de distinguer les bactéries cultivables des bactéries non cultivables.

De même, par le terme *"échantillon environnemental",* on désigne aussi bien le sol et les sédiments que les végétaux, les insectes, par exemple les arthropodes, les mollusques, les éponges, les crustacés, les plathelmintes, les nématodes, les bioréacteurs tels que les biofilms, fermenteurs, boues activées et plus généralement les milieux aquatiques mais également des échantillons biologiques animaux (par exemple le lisier de porc, bol alimentaire du rumen) ou humains et de manière avantageuse toute fraction obtenue à partir de ces environnements présentant un avantage quantitatif (forte concentration bactérienne) ou qualitatif (spécificité de la communauté ou population bactérienne).

Dans la suite de la description, le procédé objet de l'invention est plus particulièrement décrit en relation avec l'extraction d'ADN de la flore bactérienne non cultivable contenue dans les drosophiles, le sol, et les fèces d'origine humaine.

Ainsi, dans une première forme de réalisation, l'échantillon environnemental est un insecte. En effet, parmi les organismes non cultivables présentant un intérêt potentiel, sont également connues les bactéries endosymbiotes, et notamment celles du genre *Wolbachia*. Ce type d'endosymbiotes est présent chez bon nombre d'invertébrés et notamment d'arthropodes, en particulier chez les drosophiles. On retrouve également ces bactéries chez les mollusques. Plus particulièrement, les bactéries du genre *wolbachia* sont préférentiellement localisées dans les organes reproducteurs des insectes comme décrit par Werren JH. 1997. Biology of *wolbachia*.Annual Review of Entomology. 42, 587-609. Pour extraire exclusivement l'ADN bactérien plutôt que l'ADN nucléaire, le procédé de l'invention est conduit à partir d'oeufs non fécondés de drosophiles donc de femelles vierges.

Par ailleurs, le Demandeur a constaté que dans le cas des insectes et plus particulièrement de la drosophile, un broyage ménagé suivi d'une filtration puis d'une centrifugation permettait d'optimiser la séparation ultérieure de la fraction majoritairement bactérienne du reste du broyât.

Dans une deuxième forme de réalisation de l'invention, l'échantillon environnemental est un échantillon de sol ou une sous-fraction granulométrique comme par exemple décrit par Hattori T. 1988. Soil aggregates as microhabitats of microorganisms. Biology and Fertility of Soils. 6, 189-203, et par Jocteur-Monrozier L, Ladd JN, Fitzpatrick RW, Foster R.C., Raupach M. 1991. Components and microbiomass content of size fractions in soils of contrasting aggregation. Geoderma, 49, 37-62.

Selon un exemple de réalisation avantageux mais non limitatif, le sol utilisé est une fraction de la couche superficielle du sol.

Dans une première forme d'exécution, le broyage de l'échantillon de sol peut être effectué de manière préférentielle mais non exclusive dans un tampon de saccharose dont la concentration est égale à 30 millimoles par litre, d'EDTA dont la concentration est égale à 25 millimoles par litre et de TES dont la concentration est égale à 50 millimoles par litre. On entend par broyage, toutes méthodes qui permettent de disperser les cellules bactériennes des autres constituants.

Par ailleurs, l'étape de broyage comprend successivement :
◆ un premier broyage énergique de l'échantillon;
◆ une centrifugation de lavage;
◆ la reprise du culot dans une solution tampon physiologique;
◆ deux broyages énergiques complémentaires.

Bien entendu, les conditions de broyage et de centrifugation sont dépendantes de la masse de matériel de départ mise en oeuvre. S'agissant plus particulièrement de la centrifugation, elle est conduite à une vitesse comprise entre 5 000 et 10 000 rpm, avantageusement 6 000 rpm, pendant 5 à 15 minutes, avantageusement 10 minutes.

Le Demandeur a constaté que de façon tout-à-fait surprenante, cet enchaînement d'étapes permettait d'optimiser la séparation ultérieure des organismes non cultivables du reste du broyât.

Dans un second mode d'exécution, le broyage de l'échantillon de sol est effectué dans une solution tampon de NaCl à 0,8%.

Dans une troisième forme de réalisation, l'échantillon environnemental se présente sous forme d'un échantillon biologique humain ou animal. Dans un mode de réalisation avantageux, l'échantillon biologique est constitué par des fèces d'origine humaine.

Pour permettre de séparer la flore intestinale de la matière solide, le broyage est suivi d'une étape de filtration. De même que précédemment, par broyage on entend tous système de dispersion des micro-organismes.

S'agissant des milieux aquatiques, une concentration préalable de l'échantillon pourra être rendue nécessaire en fonction de son volume initial. Notamment, une approche préférée sera l'utilisation des techniques de centrifugation en flux continu comme décrit par exemple dans Centrifugation a practical approach (2 nd). 1984. Eds, D Rickwood &B.D. Hames (IRL Press, Oxford, Washington DC)

Pour tous ces échantillons environnementaux décrits de façon non limitative, la séparation des organismes non cultivables du reste du broyât est effectuée comme déjà dit, par sédimentation sur coussin de densité supérieur à 1. En pratique, on utilise au choix une solution de Nycodenz, Percoll, Ficoll, Metrizamide, Iodixanol, que l'on positionne au fond d'un tube sous le broyât. La sédimentation différentielle s'établit au fond du tube pour les particules très denses et de façon ordonnée sur le coussin pour les autres. Comme déjà dit, cette sédimentation peut être accélérée par centrifugation.

Selon une caractéristique avantageuse du procédé de l'invention, la fraction contenant les organismes récupérés à l'issue de l'étape de sédimentation est soumise à deux étapes de centrifugation ultérieures successives de lavage par l'eau, à une vitesse comprise entre 6 000 et 10 000 rpm et une température comprise entre 3 et 5° C, avantageusement 4° C.

Par ailleurs, pour permettre d'extraire le maximum d'ADN, le mélange constitué par les organismes cultivables et non cultivables inclus dans un bloc d'agarose à l'issue des deux étapes ultérieures de centrifugation est soumis à deux lyses successives :
- tout d'abord une première lyse dans un tampon à base de lysosyme et d'achromopeptidase ;
- puis, une seconde lyse dans un tampon à base de lauryl-sarcosyl à 1 % et de protéinase.

Pour permettre la séparation des fragments d'ADN, le bloc d'agarose incorporant les organismes lysés est soumis à une électrophorèse en champs alternés en gel d'agarose.

En pratique, les gels d'agarose sont des gels ultrapurs low melting point (high molecular grade) à une concentration de 0,8% (0,7 à 1,2%) de manière avantageuse. De manière préférentielle mais non limitative, les migrations électrophorétiques sont conduites sous une tension de 150V pendant des durées variables de l'ordre de 10 à 24 heures. La variation des impulsions (par exemple de 1 à 5 secondes jusqu'à 10 à 25 secondes) permettent d'obtenir les séparations optimales selon la taille des fragments d'ADN désirés, de contrôler et d'optimiser la séparation des fragments d'ADN désirés selon la taille.

Comme déjà dit et dans une forme de réalisation préférée, on soumet les brins d'ADN séparés et purifiés à l'issue de l'étape d'électrophorèse (première migration électrophorétique) en champs alternés, à une étape de restriction enzymatique suivie d'au moins une électrophorèse (seconde migration électrophorétique).

Dans ce cas, la première migration électrophorétique est utilisée pour extraire les fragments d'ADN de grande taille et les séparer des débris cellulaires. Des conditions électrophorétiques très séparatrices peuvent être avantageusement utilisées comme par exemple des impulsions de 10 à 25 secondes pendant 20 heures (150V). Cette migration préparative permet la récupération de fragments d'ADN supérieure à 300 Kb.

L'ADN d'une taille supérieure à 300 Kb contenu dans l'agarose est découpé et soumis à une restriction enzymatique dans l'agarose. Les quantités d'enzymes utilisées ainsi que le temps d'application dépendent notamment de l'activité propre de l'enzyme et de la quantité d'ADN en présence.

Dans un premier mode de réalisation, le nombre d'électrophorèses est au nombre de deux.

Dans ce cas, les blocs d'agarose, contenant les ADNs digérés, sont à nouveau positionnés dans un gel d'agarose et soumis à une ou plusieurs électrophorèses en champs alternés pour séparer et purifier les ADNs digérés dans le même gel. Avantageusement, on soumet les ADNs digérés à deux électrophorèses en champ alterné correspondant à une double migration électrophorétique. Dans un champs électrique homogène et constant, le gel est positonné alternativement dans un sens et dans le sens strictement opposé. Au cours de la première migration électrophorétique le sens du champs électrique permet de faire sortir les petits fragments d'ADN hors du gel. En effet, le positionnement excentré des blocs d'agarose permet, du coté le plus court, de faire sortir les petits fragments d'ADN tout en maintenant les grands fragments dans le gel. Dans une deuxième migration, le gel est positionné dans l'autre sens et les grands fragments d'ADN peuvent ainsi être séparés selon leur taille. Les conditions de la première migration sont de manière préférentielle 10/25 secondes pendant 4 heures. Les conditions de la deuxième migration sont de manière préférentielle de 10/25 secondes pendant 20 heures.

Dans un second mode de réalisation, le nombre d'électrophorèses est au nombre de trois.

Dans ce cas, une seconde migration électrophorétique séparatrice, dans un gel constitué de 100 ml d'agarose ultrapur low melting point (Biorad) à 0,8%, permet de séparer les fragments d'ADN digérés selon leur taille (par 5/15 secondes pendant 17h sous une tension de 150V).

On concentre les fragments d'ADN de la taille désirée en reprenant le bloc d'agarose et en le soumettant à une migration retour (troisième migration) (positionnement du bloc d'agarose en sens inverse sur un nouveau gel). Le gel et les conditions de migration électrophorétiques doivent être absolument identiques.

Une dernière migration (quatrième migration) électrophorétique est effectuée afin d'éliminer les petits fragments d'ADN enchevêtrés dans les fragments d'ADN de taille souhaitée. Cette électrophorèse est conduite dans des conditions très peu séparative n'affectant que les petits fragments (impulsions de 1/5 secondes pendant 10 heures sous une tension de 150V).

Le fait de maintenir les fragments d'ADN dans l'agarose, et de n'utiliser ni intercalants ni visualisation aux UVs permet de préserver la qualité et l'intégrité d'ADN de grande taille.

Comme déjà dit, l'invention concerne également l'ADN de taille supérieure à 300 kB, susceptible d'être obtenu par le procédé ci-avant décrit. L'ADN ainsi obtenu peut présenter, selon les différents modes de réalisation du procédé, des extrémités "cohésives" (lorsqu'il a fait l'objet de restriction enzymatique), des extrémités "bouts francs" (lorsqu'il a fait l'objet d'une cassure mécanique et de réparation enzymatique de type Klenow ou T4 DNA polymérase par exemple) ou enfin des queues homopolymériques (lorsqu'il a fait l'objet d'un traitement par l'enzyme Terminal transférase). Toutes ces techniques sont déjà largement documentées (Current protocols in molecular biology, Eds F.M. Ausubel, R. Brent, R.E. Kingston, D.D. Moore, J.G. Seidman, J.A. Smith et K. Struhl.; Published by Greene Publisbing Associates and Wiley-Interscience. Cet ADN pouvant être utilisé dans toutes expérimentations de biologie moléculaire, comme par exemple et de manière non limitative, pour la construction de librairies d'ADN en utilisant toutes les techniques de clonage adaptées, connues de l'homme du métier.

Etant donné la pureté et la taille de l'ADN, tous types de vecteurs de clonage ou d'expression peuvent être utilisés. Par exemple et de manière non limitative, ces vecteurs sont de types:
- Phagemide: pBluescript SK+ (pSK+) (Henne A., Daniel R., Schmitz R.A. and Gottschalk G, 1999 - Construction of environmental DNA librairies in *Escherichia coli* and screening for the presence of genes conferring utilization of 4-Hydroxybutyrate., Applied and Environmental Microbiology.,65:3901-390
- Vecteurs dérivés de phage: lambda ZAPII Cottrell M.T., Moore J.A. and Kirchman D.L., 1999 - Chitinases from uncultured marine microorganisms., Applied and Environmental Microbiology, 65:2553-2557
- Cosmid vecteur pCPP47; Bauer D.W. and Collmer A., 1997 - Molecular cloning, chacterization, and mutagenesis of a *pel* gene from *Pseudomonas syringae* pv. *Lachrymans* encoding a member of the *Erwinia chrysanthemi* PelADE family of pectate lyases, Molecular Plant-Microbe Interactions, 10:369-379
- Fosmid pFOS1 (vecteur décrit par Kim U.J., Shizuya H., de Jong P.J., Birren B. and Simon M.I., 1992 - Stable propagation of cosmid sized human DNA inserts in a F factor based vector, Nucleic Acids Research 20(5):1083-1085) Stein J.L., Marsh T.L., Wu K.Y., Shizuya H. and deLong E., 1996 - Characterization of uncultivated prokaryotes: isolation and alalysis of a 40-kilobas-pair genome fragment from a planktonic marine archaeon, Journal of Bacteriology, 178:591-599
- PAC vecteur Dérivé du bactériophage P1 (pCYPAC-1), Ioannou P.A., Amemiya C.T., Garnes J., Kroisel P.M., Shizuya H., Chen C., Batzer M.A. and de Jong P., 1994 - a new bacteriophage P1-derived vector for the propagation of large human DNA fragments, Nature Genetics, 6:84-89
- BAC vecteur pBeloBAC11 (pSK+) (vecteur décrit par Shizuya H., Bruce Birren, Ung-Jin Kim, Valeria Mancino, Tatiana Slepak, Yoshiald Tachiiri, and Melvin I. Simon, 1992 - A bacterial cloning system for cloning large human DNA fragments., Proc. Natl. Acad. Sci., 89:8794-8797.); Shizuya, H., Birren, B., Kim, U.-J., Mancino, V., Slepak, T., Tachiiri, Y. and Simon, M., 1992 - Cloning and stable maintenance of 300-kilobase-pair fragments of human DNA in Escherichia coli using an F-factor-based vector., Proceeding of National Academy of Science, 89, 8794-8797.; Woo, S.-S., Jiang, J., Gill, B.S., Paterson, A.H. and Wing, R.A., 1994 - Construction and characterization of a bacterial artificial chromosome library of *Sorghum bicolor.,* Nucleic Acids Research, 22, 4922-4931.; Rondon, M.R., Raffel, S.J., Goodman, M.R and Handelsman, J., 1999 - Toward functional genomics in bacteria: Analysis of gene expression in Escherichia coli from a bacterial artificial chromosome library of *Bacillus cereus*, Proceeding of National Academy of Science, 96:6451-6455.
YAC vecteur pYAC4, Larin Z., Monaco A.P. and Lehrach H., 1991 - Yeast artificial chromosome libraries containing large inserts from mouse and human DNA, Proceeding of National Academy of Science, 88:4123-4127

La ligation entre les différents vecteurs possibles et les fragments d'ADN peut se faire de façon classique comme décrit par les fournisseurs selon le type d'enzyme utilisée comme par exemple (T4 DNA ligase, Sociétés Roche ou Life Technologies; Fast Link DNA ligation Kit, Société Epicentre Technologies) ou en gel comme décrit par exemple par la Société Epicentre Technologies dans le pWEB Cosmid Cloning Kit.

La transformation ou l'encapsidation (selon le vecteur utilisé) ont été largement décrites, comme par exemple dans Current Protocols in Molecular Biology, Eds F.M. Ausubel, R. Brent, R.E. Kingston, D.D. Moore, J.G. Seidman, J.A. Smith et K. Struhl.; Published by Greene Publishing Associates and Wiley-Interscience ainsi que dans les instruction des fournisseurs.

L'invention se rapporte enfin aux librairies d'ADN ainsi constituées par les fragments d'ADN obtenus par le procédé précédemment décrit et de manière nonlimitative pour leur utilisation pour des criblages moléculaires, phénotypiques ou chimiques.

L'invention et les avantages qui en découlent ressortiront mieux des exemples de réalisation suivants à l'appui des figures annexées.

La figure 1 représente le profil de migration de l'ADN bactérien extrait selon l'exemple 1 (piste 2), selon l'exemple 2 (piste 3) et l'exemple 3 (piste 4).
La figure 2 représente le profil de migration de l'ADN bactérien extrait selon l'exemple 2, à l'issue de la seconde migration (2A) et de la troisième migration (2B).

### EXEMPLE 1 : EXTRACTION D'ADN DE BACTERIES ENDOSYMBIOTES DU TYPE WOLBACHIA A PARTIR D'OEUFS DE DROSOPHILES

**I - Matériel de départ :** 10 à 20 000 oeufs non fécondés de femelles vierges de *Drosophila simulans* sont mis en solution dans environ 500 µl de NaCl 0,8 %.

L'utilisation d'oeufs non fécondés est essentielle dans la mesure où elle permet d'extraire majoritairement de l'ADN bactérien avec une charge minimale d'ADN nucléaire.

**II - Broyage du matériel** : le broyage est effectué de façon ménagée dans un microtube de 1,5 ml au moyen d'un piston stérile en polypropylène avec ajout de sable de Fontainebleau stérile. Le broyage comprend les étapes suivantes :
- broyage de 2 minutes dans la glace ;
- brève centrifugation à 4 000 rpm à une température de 4° C ;
- récupération du surnageant trouble dans un tube Ultraclear 38 ml Kontron;
- remise en suspension du culot par 500 µl de NaCl 0,8 % ;
- on répète les étapes a) à d) sur le même échantillon ;
- les surnageants des 3 broyages sont repris dans un tube ultraclear.
- le dernier culot de broyât est repris et filtré sur coton stérile par du NaCl 0,8 % qsp 20 ml ;
- on récupère le filtrat qu'on ajoute au surnageant de f/

### III - Réalisation de l'étape de sédimentation sur gradient de densité

On prépare une solution de NYCODENZ à 30 % poids/volume (15 g dans 50 ml d'eau ultrapure) correspondant à une densité de l'ordre de 1,16. On met alors en place un coussin de 10 ml de NYCODENZ dans le tube ultraclear, au moyen d'une pipette, sous la suspension issue du broyage. La sédimentation est alors accélérée pendant 30 minutes à une température de 4° C par centrifugation à 9 000 rpm au moyen d'un rotor à godet mobiles, avec frein, du type TST 2838 (KONTRON).

### IV - Traitement de la fraction bactérienne

A l'issue de l'étape de sédimentation, on observe par microscopie optique dans le tube ultraclear plusieurs couches successives de haut en bas :
- couche 1 : fraction trouble avec débris fins en suspension et ponctuations visibles au microscope correspondant à des bactéries et des mitochondries ;
- couche 2 : fraction translucide avec ponctuations visibles au microscope ;
- couche 3 : fraction blanchâtre avec de très nombreuses ponctuations comprenant des bactéries en majorité, ainsi que des mitochondries ;
- couche 4 : fraction translucide sans aucune structure visible au microscope correspondant au coussin dense de NYCODENZ ;
- couche 5 : un culot blanchatre correspondant aux débris non retenus par la filtration sur coton.

4 à 5 ml de la fraction bactérienne sont récupérés à partir de la couche 3 au moyen d'un cône stérile. Le volume récupéré est ensuite repris par 20 ml d'eau ultrapure. On procède alors à une nouvelle centrifugation à une vitesse de 9 000 rpm à une température de 4° C pendant 15 minutes avec frein, pour culotter les bactéries. On observe que les ponctuations contenues dans les couches 1 et 2 ne peuvent être culottées, ce qui suggère qu'elles comprennent des bactéries et mitochondries dégradées. Le surnageant obtenu à l'issue de la première étape de centrifugation est éliminé et le culot bactérien est repris par environ 1,5 ml d'eau ultrapure. On procède ensuite à une nouvelle centrifugation de lavage à 9 000 rpm à 4° C pendant 5 minutes. On élimine enfin le surnageant, puis on conserve le culot à -20° C.

### V - Préparation des blocs d'agarose et lyse

Le culot bactérien en sortie de NYCODENZ est repris par 100 µl de Tris EDTA TE_{10,1}, [pH 8,0]. Cette suspension bactérienne est mélangée vol/vol à 100 µl dans un bloc d'agarose low melting point (BIORAD) équilibré à 55° C. Le bloc est coulé dans des moules (plug mould BIORAD) à raison de 100 µl par moule. Les moules sont maintenus sur de la glace pendant 20 minutes. Les plugs sont délicatement démoulés pour être repris dans un tube où la lyse est effectuée.

### Première étape de lyse

Chaque bloc est repris en tube Venoject stérile 5 ml par 3 ml de tampon de lyse 1 (TE_{10,1}, [pH 8,0], lysosyme 5 mg/ml, achromopeptidase 0,5 mg/ml).
On incube 2 heures à 37° C.

### Seconde étape de lyse

On élimine le tampon de lyse 1.
On reprend chaque bloc dans 3 ml de tampon de lyse 2 (TE_{10,1}, [pH 8,0], lauryl-sarcosyl 1%, protéinase K 100 µg/ml).
On incube 2 heures à 55° C.

On effectue trois rinçages successifs des plugs de 30 minutes, dans 3 ml de TE_{10,1}, [pH 8,0].

### VI - Electrophorèse en champ alterné

On positionne chacun des blocs dans les puits d'un gel d'agarose constitué de 100 ml d'agarose low melting point (BIORAD) à 0,8 %. On soumet le gel d'agarose à une électrophorèse en champ alterné au moyen d'un dispositif Chef DR II Pulsed Field Electrophoresis System (BIORAD). On utilise un tampon de migration constitué de Tris Borate EDTA (0.5x). La migration électrophorétique est conduite sous une tension de 150V pendant 22 heures. Des impulsions de 10 à 25 secondes permettent d'obtenir deux champs électriques alternés.

On visualise et on repère ensuite la taille de l'ADN par coloration au bromure d'éthidium de la partie du gel contenant le marqueur de taille et un double de l'ADN migré. Ainsi, l'ADN utilisé par la suite n'est jamais endommagé par l'utilisation d'intercalant ou des Uvs. Après découpage du gel, une coloration dans le bromure d'éthidium et une visualisation au Uvs permet de vérifier la taille des fragments d'ADN effectivement récupérés.

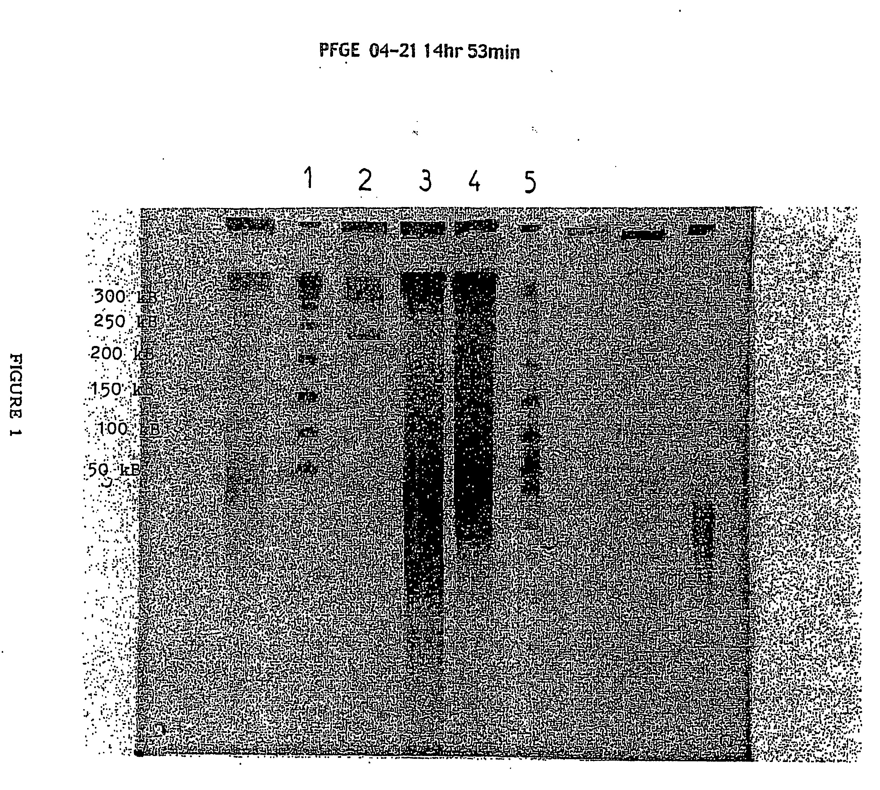

Sur la figure 1, annexée, on a représenté le profil de migration de l'ADN extrait (piste 2), les pistes 1 et 5 représentant le marqueur de taille commercialisé par la société BIOLABS sous la marque Lambda ladder PFG Marker et low range PFG marker.

Comme le montre la figure 1, le fragment d'ADN bactérien a une taille supérieure à 300 kB. Le fragment de taille compris entre 200 et 250 kB est probablement de l'ADN provenant de l'hôte drosophile. Ceci démontre bien la capacité du procédé à séparer l'ADN cible du reste de l'ADN, tout d'abord grâce au broyage et à la sédimentation différentielle sur gradient de densité (ADN génomique), puis grâce à l'électrophorèse en champ alterné (ADN mitochondrial).

### VII Récupération de l'ADN

Elle est effectuée au moyen d'une gélase (Epicentre TEBU) digérant l'agarose selon les indications données par le fournisseur
- La bande d'agarose contenant l'ADN est incubée dans trois volumes du tampon réactionnel de l'enzyme (1 heure à température ambiante);
- Le tampon est éliminé et la bande d'agarose est incubée 5 minutes à 70°C;L'agarose ainsi liquéfié est équilibré à la température de 45°C pendant 5 minutes;
- Une quantité d'enzyme de 1 à 3 unités de Gélase est ajoutée et l'ensemble est incubé 1 heure à 45°C jusqu'à complète digestion de l'agarose (la digestion est vérifiée en plongeant le tube échantillon dans la glace);
- Soit l'ADN est directement utilisé dans le milieu réactionnel de la gélase pour des réactions enzymatiques ou tout autre manipulation, soit l'ADN est précipité à l'acétate d'ammonium comme décrit par le fournisseur (Epicentre Technologies)

### EXEMPLE 2 : EXTRACTION DE LA FLORE BACTERIENNE DU SOL

**I - Matériel de départ :** 7,5 g de sol sont repris dans 50 ml d'une solution tampon comprenant Saccharose 30 mM, EDTA 25 mM, TES 50 mM

### II - Broyage du matériel

a) on broie le matériel dans un bol broyeur au moyen d'un WaringBlender pendant 1 minute à puissance maximale ;
b) on reprend en tube Falcon 50 ml et on centrifuge à 6 000 rpm à 10° C pendant 10 minutes ;
c) on élimine du surnageant et on reprend le culot par 50 ml de NaCl 0,8 % ;
d) on effectue 2 broyages de 1 minute au WaringBlender à puissance maximale avec une pause de 5 minutes entre deux dans la glace (refroidissement du broyât de sol) ;
e) on reprend le broyât en tube Falcon 50 ml et on maintient dans la glace

### III - Réalisation de la sédimentation sur coussin de densité

On prépare une solution de NYCODENZ à 60% poids/volume (30 g dans 50 ml finale d'eau ultrapure), correspondant à une densité de 1.32. On met alors en place un coussin de 10 ml de NYCODENZ dans le tube ultraclear, au moyen d'une pipette, sous la suspension issue du broyage. La sédimentation est alors accélérée pendant 10 minutes à une température de 4° C par centrifugation à 10 000 rpm, au moyen d'un rotor à godet mobile, sans frein du type TST 2838 (KONTRON).

### IV - Traitement de la fraction bactérienne

A l'issue de l'étape de sédimentation, on observe dans le tube ultraclear plusieurs couches successives de haut en bas :
- couche 1 : fraction aqueuse translucide ;
- couche 2 : fraction bactérienne blanchâtre ;
- couche 3 : coussin translucide de NYCODENZ ;
- couche 4 : sédimentation du sol

On récupère 4 à 5 ml de la fraction bactérienne de la couche 2 au moyen d'un cône stérile. On inactive ensuite les nucléases par incubation de la fraction bactérienne à 65° C pendant 10 minutes. Le volume récupéré est repris par 20 ml d'eau ultrapure. On procède ensuite à une centrifugation sur rotor à godet pour culotter les bactéries à une vitesse de 9 000 rpm à une température de 4° C pendant 20 minutes avec frein. Le surnageant est éliminé et le culot bactérien est repris par environ 1,5 ml d'eau ultrapure. On procède ensuite à une nouvelle centrifugation de lavage à 9 000 rpm à 4° C pendant 5 minutes. On élimine enfin le surnageant et on conserve le culot à -20° C.

### V - Préparation des blocs d'agarose et lyse

On répète l'exemple 1.

### VI - Electrophorèse en champ alterné

On positionne chacun des blocs lysés dans les puits d'un gel d'agarose constitué de 100 ml d'agarose low melting point (BIORAD) à 0,8 %. On soumet le gel d'agarose à une électrophorèse en champ alterné au moyen d'un dispositif Chef DR II Pulsed Field Electrophoresis System (BIORAD) pour faire une électrophorèse préparative. On utilise un tampon de migration constitué de TrisBorate EDTA (0.5x). La migration électrophorétique préparative est conduite sous une tension de 150V pendant 20 heures avec des impulsions de 10 à 25 secondes.

De même que dans l'exemple 1, L'ADN extrait à l'issue de la première migration est visualisé et repéré sur gel (voir piste 3 de la figure 1)

Les blocs d'agarose (environ 100 mg) sont alors récupérés et soumis à une restriction enzymatique par *Hind*III:
- incubation 1 heure dans 500 µl de tampon de l'enzyme (1X) à 4°C. remplacement du tampon par 250 µl du même tampon avec 10U d'enzyme;
- incubation 2 heure dans la glace
- transfert à 37°C et incubation pendant 1heure.

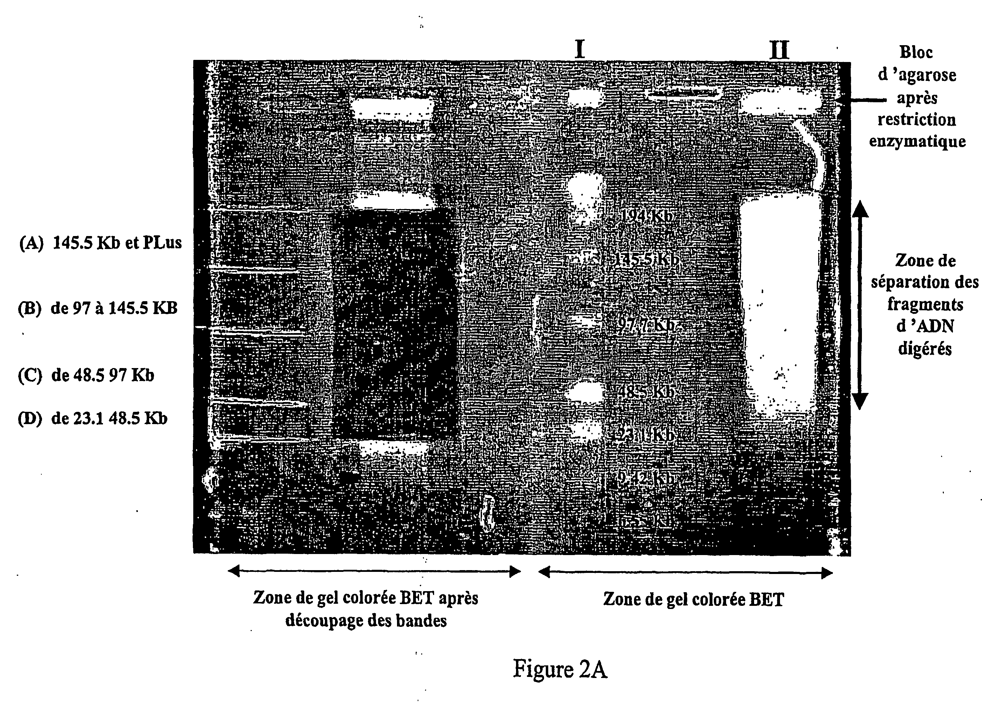

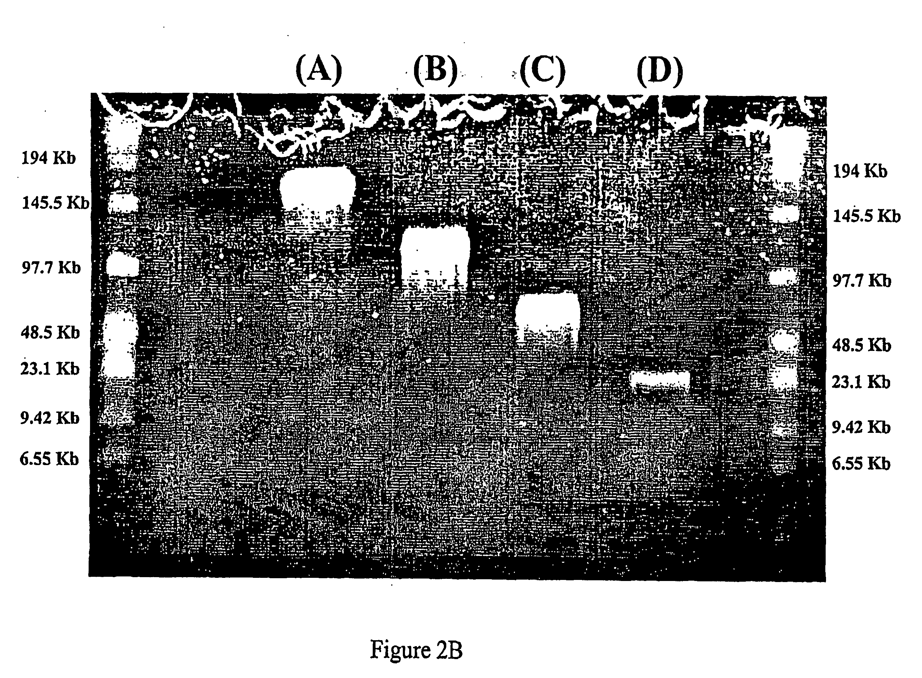

On reprend le bloc d'agarose contenant l'ADN digéré dans un gel d'agarose constitué de 100 ml d'agarose de Low Melting Point (Biorad) à 0,8%. On effectue ensuite une seconde migration électrophorétique en champs pulsés sous une tension de 150Và 5/15 secondes pendant 17 heures. Le "smear" de digestion est répéré (Fig 2A);comme décrit ci-avant et des zones de gel sont découpées selon la taille désirée. Les blocs sont ensuite disposés en sens inverse dans un nouveau gel strictement identique et soumis à une troisième migration électrophorétique en champs pulsés dans des conditions strictement identiques (concentration). Les fragments d'ADN contenus dans les différentes bandes découpées et concentrés par la troisième migration sont visualisés (Fig 2B). Les fragments d'ADN concentrés sont ensuite soumis à une quatrième migration électrophorétique sous tension de 150V, 1/5 secondes pendant 10 heures.

### VII - Récupération de l'ADN

On répète l'exemple 1.

### EXEMPLE 3 : EXTRACTION DE LA FLORE BACTERIENNE INTESTINALE

**I - Matériel de départ :** on reprend 3 g de selles dans 50 ml de NaCl 0,8 %

**II - Broyage du matériel :** le broyage est effectué au moyen d'un potter téflon stérile dans un cylindre en verre borosilicaté ;
a) broyage de 2-5 minutes dans la glace ;
b) dans l'hypothèse où le broyage au potter ne suffirait pas, on soumet la matière à un broyage très rapide en WaringBlender (5-10 secondes) ;
c) on filtre le broyât sur de la gaze stérile.

### III - Réalisation de la sédimentation sur coussin de densité

On prépare une solution de NYCODENZ à 30% poids/volume (15 grammes dans 50 ml finale d'eau ultrapure) correspondant à une densité de 1,16. On met alors en place un coussin de 10 ml de NYCODENZ dans le tube ultraclear, au moyen d'une pipette, sous la suspension issue du broyage. La sédimentation est alors accélérée pendant 30 minutes à une température de 4° C par ultracentrifugation à 9 000 rpm.

### IV - Traitement de la fraction bactérienne

A l'issue de l'étape de sédimentation, on observe dans le tube ultraclear plusieurs couches successives de haut en bas :
- couche 1 : fraction aqueuse très colorée ;
- couche 2 : fraction bactérienne blanchâtre ;
- couche 3: coussin translucide de NYCODENZ ;
- couche 4 : culot correspondant aux débris organiques denses.

On récupère environ 4 à 5 ml de la fraction bactérienne contenue dans la couche 2 au moyen d'un cône stérile. On reprend le volume récupéré par 20 ml d'eau ultrapure. On procède ensuite à une centrifugation par rotor à godet pour culotter les bactéries à une vitesse de 9 000 rpm à une température de 4° C pendant 20 minutes avec frein. Le surnageant est éliminé et le culot bactérien repris par environ 1,5 ml d'eau ultrapure. On procède ensuite à une nouvelle centrifugation de lavage à 6 000 rpm et 4° C. On élimine le surnageant et on conserve le culot à -20° C.

### V - Préparation des blocs d'agarose et lyse

On répète l'exemple 2.

### VI - Electrophorèse en champ alterné

On répète l'exemple 2.

Sur la figure 1 annexée, on a représenté le profil de migration de l'ADN extrait (piste 4). On observe que la taille du fragment d'ADN extrait est supérieure à 300 kB.

### VII - Récupération de l'ADN

On répète l'exemple 1.

## Revendications

1. Procédé d'extraction indirecte d'ADN cible d'organismes non cultivables contenus dans un échantillon environnemental selon lequel :
• on broie en milieu liquide ledit échantillon ;
• on récupère le broyât obtenu ;
• on sépare les organismes du reste du broyât par sédimentation sur coussin de densité supérieure à 1 ;
• on récupère les organismes isolés à la surface du coussin que l'on inclut dans un bloc d'agarose ;
• on extrait l'ADN par lyse dans le bloc d'agarose ;
• on positionne le bloc dans un gel d'agarose que l'on soumet à au moins une électrophorèse en champs alternés,
• enfin, on récupère les brins d'ADN extraits.

2. Procédé selon la revendication 1, **caractérisé en ce qu'**on soumet les brins d'ADN séparés et purifiés à l'issue de l'étape d'électrophorèse en champ alterné (première migration électrophorétique), à une étape de restriction enzymatique suivie d'au moins une électrophorèse en champ alterné (seconde migration électrophorétique).

3. Procédé selon la revendication 2, **caractérisé en ce que** l'étape de restriction enzymatique est suivie de deux électrophorèses en champs alternés (seconde et troisième migration électrophorétique).

4. Procédé selon la revendication 3, **caractérisé en ce que** les seconde et troisième migrations sont conduites sur un même gel, l'orientation de migration des fragments d'ADN entre la seconde et la troisième migration étant inversée.

5. Procédé selon la revendication 2, **caractérisé en ce que** l'étape de restriction enzymatique est suivie de trois électrophorèses en champs alternés (respectivement seconde, troisième et quatrième migration électrophorétique).

6. Procédé selon la revendication 5, **caractérisé en ce que** les seconde et troisième migrations sont conduites sur deux gels différents, l'orientation de migration des fragments d'ADN entre la seconde et la troisième migration étant inversée.

7. Procédé selon la revendication 6, **caractérisé en ce que** le sens d'orientation de la quatrième migration est identique à celui de la troisième migration.

8. Procédé selon la revendication 1, **caractérisé en ce qu'**on récupère les brins d'ADNs par électroélution ou digestion de l'agarose.

9. Procédé selon la revendication 1, **caractérisé en ce que** l'échantillon environnemental est un insecte.

10. Procédé selon la revendication 9, **caractérisé en ce que** l'ADN est extrait à partir d'oeufs non fécondés de femelles vierges de drosophile.

11. Procédé selon la revendication 1, **caractérisé en ce que** l'échantillon environnemental est un échantillon de sol, une sous-fraction granulométrique ou une fraction de la couche superficielle du sol.

12. Procédé selon la revendication 1, **caractérisé en ce que** l'échantillon environnemental se présente sous forme d'un échantillon biologique humain ou animal.

13. Procédé selon la revendication 12, **caractérisé en ce que** l'échantillon biologique se présente sous forme de fèces d'origine humaine.

## Patentansprüche

1. Verfahren zur indirekten Extraktion von Ziel-DNA aus in einer Umweltprobe enthaltenen, nicht kultivierbaren Organismen, gemäß dem:
• die genannte Probe in einem flüssigen Medium zerkleinert wird;
• das erhaltene Zerkleinerungsgut gewonnen wird;
• die Organismen vom Rest des Zerkleinerungsguts durch Sedimentierung auf ein Kissen mit einer Dichte von mehr als 1 getrennt werden;
• die Organismen rückgewonnen werden, die an der Oberfläche des Kissens isoliert sind, das in einen Agarose-Block eingeschlossen ist;
• die DNA durch Lyse in dem Agarose-Block extrahiert wird;
• der Block in einem Agarosegel angeordnet wird, das mindestens einer Wechselfeld-Elektrophorese unterzogen wird,
• schließlich die extrahierten DNA-Stränge gewonnen werden.

2. Verfahren nach Anspruch 1, **dadurch gekennzeichnet, daß** die abgetrennten und gereinigten DNA-Stränge am Ende des Wechselfeld-Elektrophorese-Schritts (erste elektrophoretische Migration) einem Schritt der enzymatischen Restriktion, gefolgt von mindestens einer Wechselfeld-Elektrophorese (zweite elektrophoretische Migration), unterzogen werden.

3. Verfahren nach Anspruch 2, **dadurch gekennzeichnet, daß** auf den Schritt der enzymatischen Restriktion zwei Wechselfeld-Elektrophoresen (zweite und dritte elektrophoretische Migration) folgen.

4. Verfahren nach Anspruch 3, **dadurch gekennzeichnet, daß** die zweite und dritte Migration auf demselben Gel ausgeführt werden, wobei die Migrationsrichtung der DNA-Fragmente zwischen der zweiten und der dritten Migration umgekehrt ist.

5. Verfahren nach Anspruch 2, **dadurch gekennzeichnet, daß** auf den Schritt der enzymatischen Restriktion drei Wechselfeld-Elektrophoresen (zweite, dritte bzw. vierte elektrophoretische Migration) folgen.

6. Verfahren nach Anspruch 5, **dadurch gekennzeichnet, daß** die zweite und dritte Migration auf zwei verschiedenen Gelen ausgeführt werden, wobei die Migrationsrichtung der DNA-Fragmente zwischen der zweiten und der dritten Migration umgekehrt ist.

7. Verfahren nach Anspruch 6, **dadurch gekennzeichnet, daß** die Orientierungsrichtung der vierten Migration mit der der dritten Migration identisch ist.

8. Verfahren nach Anspruch 1, **dadurch gekennzeichnet, daß** die DNA-Stränge durch Elektroelution oder Digestion der Agarose gewonnen werden.

9. Verfahren nach Anspruch 1, **dadurch gekennzeichnet, daß** die Umweltprobe ein Insekt ist.

10. Verfahren nach Anspruch 9, **dadurch gekennzeichnet, daß** die DNA aus unbefruchteten Eiern nicht begatteter weiblicher Fruchtfliegen extrahiert wird.

11. Verfahren nach Anspruch 1, **dadurch gekennzeichnet, daß** die Umweltprobe eine Bodenprobe, eine Korngrößen-Unterfraktion oder eine Fraktion der Oberflächenschicht des Bodens ist.

12. Verfahren nach Anspruch 1, **dadurch gekennzeichnet, daß** die Umweltprobe die Form einer menschlichen oder tierischen biologischen Probe aufweist.

13. Verfahren nach Anspruch 12, **dadurch gekennzeichnet, daß** die biologische Probe die Form von Kot menschlichen Ursprungs aufweist.

## Claims

1. A method for indirectly extracting target DNA of non-cultivatable organisms contained in an environmental sample, according to which :
• said sample is ground in liquid medium;
• the ground material obtained is recovered;
• the organisms are separated from the rest of the ground material by sedimentation on a cushion with a density greater than 1;
• the isolated organisms at the surface of the cushion are recovered and are embedded in a block of agarose;
• the DNA is extracted by lysis in the block of agarose;
• the block is placed in an agarose gel which is subjected to at least one alternating field electrophoresis;
• finally, the DNA strands extracted are recovered.

2. The method as claimed in claim 1, **characterized in that** the DNA strands separated and purified at the end of the alternating field electrophoresis step (first electrophoretic migration) are subjected to an enzymatic restriction step followed by at least one alternating field electrophoresis (second electro-phoretic migration).

3. The method as claimed in claim 2, **characterized in that** the enzymatic restriction step is followed by two rounds of alternating field electrophoresis (second and third electrophoretic migration).

4. The method as claimed in claim 3, **characterized in that** the second and third migrations are carried out on the same gel, the direction of migration of the DNA fragments between the second and third migration being reversed.

5. The method as claimed in claim 2, **characterized in that** the enzymatic restriction step is followed by three rounds of alternating field electrophoresis (second, third and fourth electrophoretic migration, respectively).

6. The method as claimed in claim 5, **characterized in that** the second and third migrations are carried out on two different gels, the direction of migration of the DNA fragments between the second and third migration being reversed.

7. The method as claimed in claim 6, **characterized in that** the direction of the fourth migration is identical to that of the third migration.

8. The method as claimed in claim 1, **characterized in that** the DNA strands are recovered by electroelution or digestion of the agarose.

9. The method as claimed in claim 1, **characterized in that** the environmental sample is an insect.

10. The method as claimed in claim 9, **characterized in that** the DNA is extracted from unfertilized eggs from drosophila virgin females.

11. The method as claimed in claim 1, **characterized in that** the environmental sample is a soil sample, a granulometric subfraction or a superficial soil layer fraction.

12. The method as claimed in claim 1, **characterized in that** the environmental sample is in the form of a human or animal biological sample.

13. The method as claimed in claim 12, **characterized in that** the biological sample is in the form of feces of human origin.
